# EUROPEAN PATENT SPECIFICATION

(11) **EP 0 310 251 B1**
(45) Date of publication and mention of the grant of the patent: **29.06.1994**
(21) Application number: 88308164.8
(22) Date of filing: 02.09.1988
(51) Int. Cl.: C12Q 1/68

(54) **DNA detection system**
System zum Detektierung von ONS
Système pour détecter l'ADN

(30) Priority: 04.09.1987 US 93361
(43) Date of publication of application: 05.04.1989
(73) Proprietor: Molecular Devices Corporation, Palo Alto California 94304 (US)
(72) Inventor: Kung, Viola T., Menlo Park, CA 94025 (US); Nagainis, Peter A., San Jose, CA 95120 (US); Sheldon III, Edward L., Menlo Park, CA 94025 (US)
(74) Representative: Harrison, David Christopher

(56) References cited:
- EP-A- 0 135 159
- EP-A- 0 147 665
- WO-A-85/05685
- FR-A- 2 606 154
- GB-A- 2 125 964
- US-A- 4 493 899
- BIOLOGICAL ABSTRACTS, vol. 83, 1986; M.I. KHAMIS et al., no. 83033298#
- CHEMICAL ABSTRACTS, vol. 98, no. 17, 25 April 1983, Columbus, OH (US); J.V. TRICOLI et al., p. 238, no. 139462u#
- BIOCHEMISTRY, vol. 25, no. 1, 1986, Easton, PA (US); T.M. LOHMAN et al., pp. 21-25#

## Description

This invention relates to methods for detecting the presence of DNA. The method employs high affinity binding proteins for single stranded DNA.

The amount of DNA in a sample has traditionally been measured either by spectrophotometric means or fluorometrically with the use of ethidium bromide. If the sample is pure (does not contain significant amounts of contaminants such as other nucleic acids, protein, phenol, or agarose) the spectrophotometric measurement of the amount of ultraviolet (UV) irradiation absorbed is simple and accurate. However, if there is contamination with protein or compounds which absorb strongly in the UV, such as phenol, accurate quantitation of the amount of DNA will not be possible. Furthermore, this technique is only suitable for samples containing DNA in the »g/ml range.

If the amount of DNA in the sample is small, or if the sample contains significant quantities of impurities, the amount of DNA may be estimated from the intensity of UV-induced fluorescence emitted by ethidium bromide intercalated into the DNA. The amount of fluorescence is proportional to the total amount of DNA. The quantity of DNA in the sample therefore can be estimated by comparing the fluorescent yield of the sample with that of a series of standards. As little as 1 to 5 »g/ml of DNA can be detected by this method. With the use of a mini-fluorometer (such as that manufactured by Hoefer Scientific Instruments, San Francisco, CA) and the fluorochrome Hoechst 33258, the sensitivity may be increased to 10 ng/ml.

With the advent of recombinant DNA technology, it has become imperative to be able to identify significantly lower concentrations of DNA in a sample, for example, any contaminating DNA which may be present in a recombinant product. The contaminating DNA may be non-specific and of unknown sequence. Therefore, enzyme amplification of sample DNA (using for example the DNA polymerase chain reaction method) is difficult for lack of universal primers for DNA synthesis. There is, therefore, substantial interest in being able to detect rapidly and accurately the presence of extremely small amounts of DNA.

### Relevant Literature

Krauss et al., Biochemistry (1981) 22:5346-5352 disclose the binding of single-stranded binding proteins from E. coli to oligonucleotides. Vogelstein and Gillespie, Proc. Natl. Acad. Sci. USA (1979) 76:615-619 disclose the binding of DNA to glass. Kung et al. disclose the purification of topoisomerase I from Micrococcus luteus by high salt elution from a DNA-sepharose column; J. Biol. Chem. (1977) 252:5398-5402. The following are review articles pertaining to DNA binding proteins. Gellert, The Enzymes, Vol. XIV (1981) 345-366; Wang, The Enzymes, Vol. XIV (1981) 332-343; Chase, Ann. Rev. Biochem. (1986) 55:103-136; Kowalczykowski et al., The Enzymes, Vol. XIV (1981) 375-444.

### SUMMARY OF THE INVENTION

A novel method is provided for detecting the presence of DNA in a sample, which employs a DNA-detection system in which one of the components is a DNA binding protein which has high affinity for single-stranded DNA (ssDNA). To detect the presence of DNA, a sample, optionally pretreated if it contains protein, is denatured to yield ssDNA. The sample is then contacted with a high-affinity single-stranded DNA binding protein (BP) to form ssDNA-BP complexes which may then be detected by means of a label bound to either the ssDNA or the BP. The BP and the ssDNA may both be in solution, at the time of contacting, or either of the ssDNA or the BP may be bound non-diffusively to a solid support prior to the time of contacting. When bound to the solid support, the ssDNA or the BP may be bound directly to the solid support or bound by means of a linker molecule. The method may be used to detect DNA in, for example, recombinant protein products, or to detect contaminating organisms in a sample.

### DESCRIPTION OF THE SPECIFIC EMBODIMENTS

In accordance with the subject invention, methods and compositions are provided for detecting the presence of DNA in a sample, particularly in a protein product produced in culture, e.g., fermentation. The presence of DNA may be detected by the use of high affinity single-stranded DNA binding proteins.

The high affinity single-stranded DNA binding protein (BP) may be from any source, either eukaryotic or prokaryotic, and may include single-stranded DNA binding proteins, topoisomerases, and DNA unwinding proteins. Of particular interest are single-stranded DNA binding protein from E. coli (SSB), T4 gene 32 protein and topoisomerase I from Micrococcus luteus and E. coli. Methods of isolation include affinity chromatography as described in Lohman et al., Biochemistry (1986) 25:21-25. BP is also commercially available, for example, from United States Biochemical Corporation, Cleveland, Ohio.

The DNA binding protein is characterized as having high affinity for single-stranded DNA (ssDNA), at least 10⁵ M⁻¹, usually in the range of about 10⁸-10¹⁰ M⁻¹ when the ssDNA is at least 100 nucleotides long. Alternatively, the protein may be characterized as a single-stranded DNA binding protein requiring a concentration of greater than about 0.4 M sodium chloride (or other monovalent salt providing comparable ionic strength) for elution from ssDNA-cellulose or ssDNA-sepharose. Generally the concentration for elution is greater than about 0.6 M sodium chloride, and preferably greater than about 1.0 M sodium chloride. When determining the affinity, an aqueous buffer, pH 6 to 9 at 25°C should be used. No detergent, denaturant (for example, urea, guanidium chloride), chaotropic agent or organic solvent should be present in the buffer.

The BP can be used unbound to any other component, and/or it can be non-diffusively bound, covalently or adsorptively, to a solid support either directly or through a linker molecule or covalently to a label. When a linker molecule is used, the BP is bound to one half of a specific binding pair, the other half being the linker molecule. Examples of specific binding pairs include biotin and anti-biotin; avidin and streptavidin.

Various solid support materials may be employed. Thus the solid support may include filter membranes, preferably Immobilon™ or nitrocellulose. For nitrocellulose membranes, the pore size of the membrane is less than 5 »m, preferably less than 1 »m; and is usually greater than 0.05 »m, preferably greater than 0.1 »m. Conventional procedures as appropriate are used for non-diffusive binding of the BP to the solid support. Methods include covalent binding to carbonyl imidazole groups, or other active groups present on the membrane, or non-covalently by adsorption to the membrane.

The solid support material may also include chromatographic bed materials, monodisperse latex particles, including those based on styrene, chemically-modified styrene, propylene, methacrylate, butadiene, acrylonitrile or other monomers; polyglutaraldehyde microspheres (e.g., as manufactured by Polysciences, Inc.), nylon beads, chemically-modified nylon beads, oxirane acrylic beads such as Eupergit® (Rohm Pharma, Darmstadt, W. Germany); copolymers of acrylic ester and acrylic amide. Methods of binding BP to these materials include the following: BP may be covalently bound to an activated chromatographic resin having reactive groups capable of forming covalent bonds with proteins, such as CNBr-activated Sepharose-4B, CNBr-activated 4% Agarose or CNBr-activated Sepharose-6MB (Pharmacia P-L Biochemicals; Piscataway, NJ), or other resin, such as cellulose, by conventional means. BP may be bound to polystyrene beads by non-specific adsorption. BP may also be bound covalently to polystyrene beads containing carboxyl or amino functional groups (Polysciences, Inc.; Warrington, PA) by conventional means.

The DNA, generally denatured to single-stranded DNA (ssDNA), can be bound non-diffusively to a solid support, either absorptively or covalently, either directly or through a linker molecule. The solid support can be a membrane such as nitrocellulose. The sample containing the ssDNA is preferably filtered onto the membrane. To facilitate binding to the membrane, the salt concentration of the sample is generally greater than 50 mM sodium chloride, preferably greater than 100 mM sodium chloride, or other salt providing similar ionic strength. The ssDNA is then fixed on the membrane by, for example, baking the membrane at between 75°C and 100°C, preferably at 80°C for at least 30 minutes, usually for at least 1 hour, and preferably no more than 6 hours. Other methods of fixing the ssDNA on the membrane include washing the membrane with ethanol.

When the DNA is bound to the solid support through a linker molecule, the linker molecule may be any molecule which has high affinity for DNA, such as an antibody to DNA, a BP, or the like. In a preferred method, the linker molecule comprises a conjugate of biotin and anti-DNA or biotin and BP bound non-diffusively to the solid support.

The solid support can also be positively charged nylon, such as beads or membranes (for example Nytran® (Schleicher and Schull, Inc.; Keene, NH), Gene-Screen Plus™ (duPont Company; Boston, MA), Zeta-Probe® (BioRad Labs; Pinole, CA), Bio-Trace™ (Gelman Sciences, Inc.; Ann Arbor, MI), Bio-dyne B® (Pall Biosupport; Glen Cove, NY), and Genatran™ (Plasco, Inc.; Woburn MA)). The ssDNA can be selectively non-diffusively bound to the positively-charged nylon by incubating the nylon in a buffer at between pH 6 and 9 comprising an appropriate salt concentration and/or non-ionic detergent. The appropriate salt concentration is preferably less than 1 M sodium chloride (or other salt providing similar ionic strength), and preferably less than 0.6 M sodium chloride. Examples of non-ionic detergents which can be used include Tween-20® or Triton X-100® at a concentration of 0.1-5.0% v/v.

The sample may be any sample in which it is desired to detect DNA when it is present at a low concentration. The sample may be a solid or a liquid, such as a proteinaceous lyophilized composition or aqueous medium. Samples can include proteins made by recombinant DNA methods, for example, tissue plasminogen activator, insulin, growth hormone, interleukin 2, and interferons; monoclonal antibodies prepared for therapeutic purposes; water for use in procedures requiring absolute purity. The DNA may be in the form of naked DNA, either double-stranded or single-stranded, or it may be in the form of a whole cell, either prokaryotic or eukaryotic.

The method for carrying out the subject invention generally is as follows, although other variations are within the scope of the invention.

If the DNA is contained in whole cells such as bacteria or yeast cells, the cells can be lysed by exposure to lytic conditions such as treatment with sodium hydroxide, chaotropic agents such as potassium thiocyanate and the like. If the sample contains protein, the protein is optionally removed. The DNA is then denatured to ssDNA, and the ssDNA bound non-diffusively to a solid support. If the solid support does not contain non-diffusively bound BP, the ssDNA may be fixed on the solid support by for example baking, treatment with ethanol, or other convenient means. BP is then added to the solid support and binds specifically to the ssDNA. When the solid support comprises BP, the ssDNA binds specifically to the BP on the solid support and the fixing step is omitted. Whether any DNA is present in the sample is determined by detecting ssDNA-BP complexes on the solid support by means of a label generally on either the BP or the ssDNA. Alternatively, the BP may be added directly to the denatured sample where it binds to ssDNA to form BP-ssDNA complexes. The sample is then filtered.

When it is desired to determine the concentration of DNA present, at least one background solution containing no DNA and at least one reference solution containing a known amount of DNA are treated identically to the sample containing an unknown concentration of DNA. The amount of label detectable in the background solution is subtracted from the amount of label detectable in the reference solution and the unknown sample. The adjusted values for the reference solution and the unknown sample are then related to determine the amount of DNA present in the sample.

The following are general methods for carrying out the above steps. The method of the present invention can be used for the detection of DNA in either the presence or absence of protein. When protein is present, an additional step to deproteinize the sample is desirable. Any conventional means for deproteinization can be used (for example, phenol extraction) which does not adversely affect the integrity of the DNA. If the protein has known characteristics, the sample may be deproteinized by ion-exchange column chromatography (for example, DEAE-cellulose, phosphocellulose, sulfonic gel), hydroxyapatite (the single-stranded and double-stranded DNA may be separated from protein by elution with differential salt concentrations), gel filtration, and affinity chromatography.

Affinity chromatography may be used to remove the protein directly from the sample, e.g., using immobilized mouse immunoglobulin raised against the protein to be removed, or the sample may be deproteinized using immobilized BP to bind the DNA in the sample. The DNA can then be eluted from the BP using a high-salt concentration, usually greater than 1 M, preferably greater than 2 M, and the eluant used directly in the detection assay after adjusting the salt concentration. The final salt concentration varies depending upon the protocol used. For example, where the solid support is a positively charged nylon membrane or a membrane comprising a linker molecule, the salt concentration is adjusted to isotonic. For detection of DNA in a monoclonal antibody sample, the monoclonal antibody may be removed using protein A bound to a solid support.

Other methods of deproteinizing the sample include mixing the sample with a suspension of glass particles in the presence of a high concentration of sodium iodide. Any DNA present in the sample is non-diffusively bound by the glass particles. The glass particles are isolated, and the DNA recovered from the particles by treatment with water or phosphate buffered saline (PBS). The glass particles may include finely ground glass beads, or preferably a composition comprising Glassmilk™ as supplied by BIO 101, Inc., La Jolla, California.

Another method which can be used to deproteinize the sample is admixing the protein-containing sample with a proteolytic enzyme composition comprising, for example, at least one of the enzymes pronase or proteinase K. Following the enzymatic treatment, hydrolyzed product is generally removed, for example, by centrifugation through a membrane with a low molecular weight cutoff (approximately 10,000 or 30,000 as supplied by Centriprep-10, Centriprep-30; Amicon, Danvers, MA), use of a Millipore low-volume ultrafiltration device with a low molecular weight cutoff (approximately 10,000 or 30,000) or gel filtration. After any protein present is digested, removed, or digested plus removed, the DNA is denatured to ssDNA. Methods used to denature the DNA include heating at about 90-100°C or treatment with sodium hydroxide (pH 13.0). After rapid chilling (to prevent the DNA from reannealing) or neutralization (using for example, ammonium acetate or Tris buffer), the ssDNA is contacted with a solid support.

If the solid support is a membrane such as nitrocellulose or positively-charged nylon, the sample is generally filtered using a manifold filtration device. However, if the solid support is, for example, positively-charged nylon beads, the beads can be incubated directly in the sample. When the solid support has BP immobilized on its surface, the ssDNA binds to the BP to form BP-ssDNA complexes. If the solid support does not contain BP, the DNA is fixed on the solid support by baking, or treatment with ethanol. This step can be omitted when a positively-charged nylon membrane is used.

Non-specific binding sites on nitrocellulose or positively-charged nylon can be blocked by incubation of the membrane or beads with a high-concentration protein solution such as about 1 to 10% bovine serum albumin (BSA), non-fat dry milk solution and the like. For the positively-charged nylon, the non-specific binding sites additionally can be blocked by washing with a non-ionic detergent solution, such as Tween-20® or Triton X-100®, usually 0.1-5.0%.

The solid support comprising non-diffusively bound ssDNA is then incubated with labeled BP to form BP-ssDNA complexes. When the solid support is a nitrocellulose or positively-charged nylon membrane, buffer (pH 6-9) containing BP (generally about 0.3 »g/ml) is added to the membrane. The buffer is generally at room temperature and contains sodium chloride, preferably 0.01-0.3 M, and in addition, for the positively-charged nylon, contains a non-ionic detergent such as Tween-20® or Triton X-100®, preferably 0.1-5.0% v/v.

Any BP-ssDNA complexes can be detected by means of a label attached to either the BP or the ssDNA, the label preferably being attached to the BP. An exception is when the BP is pre-attached to the solid support, when the DNA is preferably labeled. The BP can be covalently labeled in a number of ways. The label can be an enzyme, foz example, alkaline phosphatase, β-D-galactosidase, glucose-6-phosphate dehydrogenase, glucose oxidase, horseradish peroxidase, β-lactamase, urease; a radionuclide, such as ¹²⁵I; a chemiluminescent or fluorescent compound, such as fluorescein isocyanate; a hapten such as biotin, and the like; or any other label which provides a detectable signal. When the label is an enzyme such as urease, which contains at least one free, accessible, non-essential cysteine residue. BP can be coupled to the enzyme, for example, as described by Blakely et al., J. Mol. Catalysis (1984) 23:263-292). Other enzymes which can be coupled in this way include β-D-galactosidase.

Alternatively, an enzyme label can be thiolated and then conjugated to the BP. Methods for attaching labels to proteins are described in detail in the scientific literature. See for example Healey et al., Clinica Chimica Acta (1983) 134:51-58; Ishikawa et al., J. Immunoassay (1983) 4:209-327; and Tijssen, Practice and Theory of Enzyme Immunoassays (1985) 259-263, Elsevier Science Publishers [Amsterdam].

When the label on SSB is an enzyme, the number of enzyme molecules bound per SSB molecule is usually about 1 to 3 maleimide molecules/SSB, preferably 1.8 maleimides/molecule. Generally this results in an SSB-enzyme conjugate comprising 1 enzyme molecule per SSB molecule. When the label is a hapten such as biotin, the number of hapten molecules bound per SSB molecule is usually 1 to 5, preferably 3 to 4.

The DNA to be detected can be labeled, rather than the BP. The label may include a radionuclide, fluorophore or a hapten such as digoxin or biotin and the like. The label can be introduced to the DNA by any standard enzymatic reaction such as nick translation, or by terminal labeling, with ³H, ¹⁴C ³²P, or biotin-labeled deoxynucleotide triphosphates (dNTP). The labeled DNA is then denatured to ssDNA by alkali or heat.

Alternatively, the DNA can be labeled with a reagent such as isopsoralen which binds to double-stranded DNA. ³H-isopsoralen or biotin-isopsoralen is available from HRI Research, Inc., Berkeley, California. The isopsoralen reagent is bound to DNA by mixing it with a sample, followed by photoirradiation at 340-380 nm. When the label used is isopsoralen, it is unnecessary to denature the labeled DNA, as isopsoralen-labeled DNA is recognized by BP without any denaturing step.

Methods of detecting BP-ssDNA complexes will depend on the type of label used as well as the sensitivity required. When the label is an enzyme, the disappearance of substrate or appearance of reaction product may be measured spectrophotometrically following substrate addition. If the enzyme is, for example, urease, an indicator dye such as cresol red may be used to monitor the change in pH in the sample following addition of enzyme substrate. The change in optical density or the visual intensity of the color change is then correlated with the DNA content of the sample by comparison with at least one identically treated reference solution containing a known concentration of DNA. Alternatively, any DNA present may be detected by measuring the amount of pH change with a photoresponsive device such as that described in U.S. Patent No. 4,591,550. Other methods of detecting BP-ssDNA complexes in the sample may include quantitating the amount of radioactivity, when the label is a radionuclide. When the label is a hapten such as biotin, the label can be detected by, for example, the use of enzyme-labeled avidin, streptavidin or antibiotin.

For convenience, the reagents are frequently provided in kits, where they may be present in conjunction with buffer, stabilizers, excipients and the like. The kit may also include any additional reagents necessary for the preparation of labeled BP or ssDNA and the detection of the labeled BP-ssDNA complexes in the performance of the assay. Where the reagents include BP, it may be provided labeled or unlabeled. When unlabeled, it may also be provided bound non-diffusively to a solid support.

The following examples are offered by way of illustration and not by way of limitation.

### EXPERIMENTAL

### Example 1

### Preparation of BP-Enzyme Conjugates

### A. Preparation of SSB-Urease Conjugate Using m-Maleimidobenzoyl-N-hydroxysuccinimide Ester (MBS)

Single-stranded DNA binding protein from E. coli (SSB) was obtained from United States Biochemical Corp. Cleveland, OH. It was coupled to the cross-linking agent MBS as follows. One hundred »l of 0.25% MBS in dimethylformamide (DMF) was added to 2.0 ml containing 2 mg of SSB in 0.1 M phosphate buffer, pH 6.8. The mixture was stirred gently at room temperature for 30 min then separated on a Sephadex G-25. The elution buffer was 0.1 M phosphate, pH 6.8. Fractions were monitored by UV absorbance at 280 nm. The first peak eluted from the column contained SSB coupled to MBS. The peak fractions (3 ml) were combined with 4 ml of urease (20 mg) in 0.1 M phosphate buffer, pH 6.8. The mixture was stirred for 20 min at room temperature. The reaction was then stopped by the addition of 1.75 ml of 500 mM sodium sulfite, in 0.1 M sodium phosphate buffer, pH 6.8, containing 10 mM dithiothreitol (DTT). The conjugate formed was separated from unconjugated enzyme by gel filtration chromatography. The purified enzyme conjugate was then diluted 1:1 (v/v) with glycerol. BSA was added to 0.25% (w/v). The conjugate was stored at 2-8°C.

### B. Preparation of SSB-Horseradish Peroxidase (HRP) Conjugate Using m-maleimidobenzoyl-N-hydroxysuccinimide ester (MBS)

HRP (Boeringer-Mannheim, La Jolla, CA) was thiolated with N-succinimidyl 3-(2-pyridyldithio)propionate SPDP) (Pierce, Rockville, IL) by combining 16 mg HRP in 0.1 M potassium phosphate buffer, 0.2 M sodium chloride, pH 7.5 with 435 »g SPDP in 25 »l dimethylformamide (DMF), and allowing coupling to take place for 30 min at room temperature. Unreacted SPDP was removed by chromatography on Sephadex G-25, eluted with 0.1 M potassium phosphate buffer containing 0.2 M sodium chloride, pH 7.0.

Dithiopyridine groups on HRP were deblocked by adding 25 mM DTT for 30 min then removing the DTT and the 2-thiopyridine formed by G-25 separation in PBS.

Maleimido-SSB was formed by adding 25 »l of a 0.25% solution of MBS in DMF to 0.55 mg of SSB in 0.5 ml of 0.1 M sodium phosphate buffer, and stirring for 30 min at room temperature. Maleimido-SSB was purified on Sephadex G-25, then condensed with 15 mg of thiolated HRP by combining the two solutions and reacting for 20 min at room temperature. The reaction was stopped by addition of 12.5 »l of 100 mM 2-mercaptoethanol. The SSB-HRP conjugate solution was made 10% (v/v) in glycerol and stored at 4°C.

### C. Preparation of SSB-Biotin Conjugate.

One mg of SSB in PBSE (150 MM, NaCl 50 mM, sodium phosphate pH 7.0 and 1 mM EDTA) was mixed with 50 »g of biotinamidocaproate-N-hydroxysuccinimide ester in 20 »l of DMF for 2 hrs at room temperature with stirring. Unreacted biotin reagent was removed by passage over a Sephadex® G-25 column.

### D. Preparation of Anti-DNA-Urease Conjugate

One mg of purified anti-DNA monoclonal antibody, clone 4H2 (obtained from Dr. Richard Weisbart at the Sepulveda Veterans Administration Hospital, Los Angeles) in 1 ml PBSE was reacted with 125 »g of 4-(N-maleimedo methyl) cyclohexane-1-carboxolic acid N-hydroxysuccinimide ester (SMCC) in 50 »l of DMF. Unreacted SMCC was removed by passage over a Sephadex® G-25 column eluted with PBSE. The number of maleimide groups per antibody was 4. Urease (20 mg) was then added to the maleimide-antibody and incubated for 4 h at 4°C. Unreacted maleimide groups were then blocked by addition of 2-mercaptoethanol to a concentration of 2mM.

Conjugate was purified by gel filtration chromatography on a 1.5 cm x 70 cm column of Sephacryl® S-400 HR (Pharmacia, Piscataway, NJ) in a buffer composed of 50 mM sodium sulfite, 20 mM sodium dihydrogen phosphate, 200 mM sodium chloride, 1mM EDTA, 2 mM dithiothreitol and 0.1% Tween®-20. The entire protein peak eluting ahead of the free urease peak was combined. 0.05% BSA was added and the conjugate solution was stored at 4°C in sealed containers under argon gas.

### E. Preparation of Biotin-Anti-DNA Conjugate

One mg of purified anti-DNA antibody from R. Weisbart in PBSE was added to 50 »g of biotin amido caproate-N-hydroxysuccinimide ester in 20 »l DMF and stirred for 2 hrs at room temperature. Unreacted biotin reagent was removed by passage over a Sephadex® G-25 column. Purified anti-DNA-biotin was stored at 4°C.

### Example 2

### Binding of SSB to Solid Supports

### A. Immobilization of SSB on an Immobilon™ Membrane

Immobilon™ membrane contains carbonyl imidazole groups which bind to epsilon amino groups on lysine or arginine. SSB was immobilized on a 0.65 »m Immobilon™ membrane (Millipore; Bedford, MA) by soaking the membrane in phosphate buffered saline containing 0.2 mg/ml SSB at room temperature for 1 hour (10 cc protein solution/ 100 cm² membrane). The SSB solution was removed by decanting. Any remaining active carbonyl imidazole groups on the membrane were quenched with 0.1 M ethanolamine (pH 9.5) at room temperature overnight. The membrane was then washed successively in phosphate buffered saline, distilled water, and polyvinylalcohol (15 min each wash), and then dried at 65°C for 5 min. The dried membrane was then ready for use in the detection system. Four hundred »l of a sample containing single-stranded ³²P-labeled DNA (from 10-1000 pg) in a 10 mg/ml BSA aqueous solution was filtered through the SSB-Immobilon™ membrane (filtration time of 10 min). Seventy percent of the ³²P counts were captured on the membrane when the SSB concentration was 0.2 mg/ml. Increasing the SSB concentration used in the immobilization procedure to 1 mg/ml improved the DNA capture to 80%.

### B. Immobilization of SSB on Nitrocellulose Membranes

SSB was adsorbed to a nitrocellulose membrane (0.45 » pore size, Schleicher and Schuell) by soaking the membrane in PBS containing 50 »g/ml SSB at 4°C overnight. Non-specific binding sites on the membrane were blocked with 10 mg/ml BSA at room temperature for 1 hour. When 400 »l of samples containing 10 mg/ml BSA and ³²P-labeled single-stranded DNA were filtered through the membrane, 36% of the ³²P counts were captured on the nitrocellulose membrane.

### Example 3

### Detection of Pure DNA in a Sample

### A. Visual Determination

Samples containing pure calf thymus DNA (Sigma Chemical Co., St. Louis, MO) from 0-100 pg/sample (in 10 mM sodium phosphate, pH 7.0, 0.15 M sodium chloride, 1 mM EDTA) were denatured to single-stranded DNA by heating at 100°C for 10 min followed by rapid chilling. The denatured samples were filtered through 0.45 » nitrocellulose membrane (Schleicher and Schuell; Keene, NH) using a manifold filtration device. The membranes were then baked at 80°C for 1 hour to fix the DNA. SSB-urease conjugate, prepared as in Example 1A and used without further purification, was diluted to 0.3 »g/ml in 2% bovine serum albumin (BSA), 2% Ficoll, 2% polyvinylpyrrolidone, 10 mM sodium phosphate, 40 mM sodium chloride, 2 mM EDTA, pH 7.5 was added to the membrane. The membrane was incubated with the SSB-urease conjugate for 1 hour at room temperature in a Petri dish in a sufficient volume of conjugate to cover the membrane. The membrane was then washed three times with 0.15 M sodium chloride, 1 mM EDTA (pH 6) to remove any non-specifically bound conjugate. Any DNA present was then detected by the addition of enzyme substrate (100 mM urea, 0.15 M sodium chloride, 1 mM sodium phosphate, pH 6, 0.5 mM cresol red). The change in pH due to the urease reaction resulted in a color change of the cresol red from orange to purple-red. The visual intensity of the purple-red spot was then correlated with the DNA content of the sample by determining the relative size of the colored spots on the membrane and the intensity of the color.

**Table 1**

| COLORIMETRIC DETECTION OF DNA | |
|---|---|
| Pure DNA (pg/sample) | Intensity of Color at 3 min |
| 0 | - |
| 10 | + |
| 20 | ++ |
| 50 | +++ |
| 100 | ++++ |

### B. Biosensor pH determination

Any DNA present on membranes prepared as described above can also be detected by measuring the amount of pH change following addition of enzyme substrate, using a photoresponsive device (see for example U.S. Patent No. 4,591,550). An enzyme substrate mixture containing 1 mM sodium phosphate, 0.05% Tween-20, 100 mM urea, pH 6 was added to the membranes. The change in pH due to the urease reaction resulted in a change in the signal of the photoresponsive device. The change in the signal (in »volt/ sec) was then correlated with the DNA content of the sample.

**Table 2**

| DETECTION OF DNA BY BIOSENSOR pH DETERMINATION | |
|---|---|
| DNA (pg/sample) | »volt/sec |
| 0 | 64 |
| 12 | 107 |
| 25 | 153 |
| 50 | 268 |
| 100 | 341 |

### Example 4

### Preparation of Solid Supports for Specific Capture of DNA

### A. Preparation of Anti-Biotin Membrane

### 1. Nitrocellulose

Anti-biotin (Sigma) was dissolved in PBS at a concentration of 0.25 mg/ml. A nitrocellulose sheet (0.8 ») (Schleicher and Schuell) was wetted with DNA-free water, then incubated in the anti-biotin mixture (0.2 ml/cm² membrane) with gentle rocking membrane for 15 min at room temperature, then overnight at 4°C. The anti-biotin mixture was then poured off and the nitrocellulose rinsed briefly with PBS (0.2 ml/cm²). The nitrocellulose was then incubated with 0.1% (w/v) glutaraldehyde in PBS for 15 min. The nitrocellulose was then washed successively with PBS, then DNA-Free water, both at 0.2 ml/cm². The nitrocellulose was then wetted with 0.2% (w/w) polyvinyl alcohol for 10 min at 0.2 ml/cm². The nitrocellulose was then baked for 10 min at 60°C.

### 2. Cellulose acetate

An anti-biotin membrane was prepared as described in Section 3A.1, above, substituting a cellulose acetate membrane (Schleicher and Schuell, 1.2 » pore size), except that the wash with PBS prior to the glutaraldehyde/PBS incubation was omitted.

### Example 5

### Detection of DNA in Samples Containing Protein by absorption of the DNA to Nitrocellulose Membrane

### A. Protein removal by glass beads

Four hundred »l of sodium iodide (6 M) were added to 200 »l of samples, each containing 2 mg of BSA and 100, 50, 25, or 0 pg calf thymus DNA. Two »l of Glassmilk™ were added and the mixture incubated for 10 min at room temperature. The Glassmilk™/DNA complex was pelleted by centrifugation for 10 sec in a microcentrifuge. The pellet was washed with 150 »l 20 mM Tris buffer, containing 200 mM sodium chloride, 2 mM EDTA in 55% methanol. The wash procedure was repeated once. After the DNA was eluted with 400 »l phosphate buffered saline, each sample was heated at 100°C for 10 min to denature the DNA then rapidly chilled. The sample was filtered onto nitrocellulose membranes. DNA was detected using the visual determination procedure described in Example 3A.

**Table 4**

| DETECTION OF PROTEIN-CONTAINING SAMPLES | |
|---|---|
| DNA DNA (pg/sample) | Intensity of Color at 3 min |
| 0 | slight positive |
| 25 | ++ |
| 50 | +++ |
| 100 | ++++ |

### B. Proteinase digestion of protein

Proteinase K and dithiothreitol were added (final concentration 100 »g/ml and 50 mM, respectively) to 100 »l of samples each containing 1 mg BSA and 100, 50, 25, 12, or 0 pg of DNA in phosphate buffered saline. The mixture was incubated at 55°C for 2 hours to digest the protein. After digestion, all samples were heated at 100°C for 5 min to inactivate proteinase K and denature DNA to single-stranded DNA. Control samples containing a matching amount of DNA but no protein were denatured at the same time. Each sample, after rapid chilling to prevent reannealing of the DNA, was filtered through a nitrocellulose membrane. Visual determination procedures were carried out as described in Example 3A to detect the presence of DNA.

**Table 5**

| DETECTION OF PROTEINASE-DIGESTED, PROTEIN-CONTAINING SAMPLES | | |
|---|---|---|
| DNA (pg/sample) | Intensity | |
| | With Protein | Without Protein |
| 0 | - | - |
| 12 | + | + |
| 25 | ++ | ++ |
| 50 | +++ | +++ |
| 100 | ++++ | ++++ |

### Example 6

### Detection of DNA in Sample Containing Protein by Adsorption of the DNA to Positively Charged Nylon

### A. Visual Detection of DNA in Insulin Samples using SSB-HRP Conjugate

7.5 »l of proteinase K (2 ng/ml) were then added to 150 »l solution of porcine insulin (Cal Biochem, La Jolla, California) 10 mg/ml in 10 mM TRIS-HCl 1 mM EDTA, pH 8.7, ccntaining 0, 5, 10, 20, or 40 pg of double-stranded calf thymus DNA. The samples were incubated overnight at 55°C, boiled at 100°C for 5 min, then chilled on ice. The digested samples were then filtered through a positively charged nylon membrane (Genatran™, 6 cm x 8 cm obtained from Plasco, Inc., Woburn, MA) at a rate of about 100 »l/min. DNA was detected by incubating the membrane with 0.2-0.5 ml/cm² SSB-HRP conjugate (150 ng/ml in 50 mM sodium phosphate, pH 7.4; 150 mM NaCl; 2 mM EDTA, 0.1 mg/ml; 0.1 mg/ml BSA; 5% Triton-X 100®) for 40 min at room temperature in a petri dish. The membrane was then washed three times by incubating the membrane in PBS containing 1 M urea and 1% dextran sulfate for 3 min each wash to remove any non-specifically bound SSB-HRP. The membrane was then washed with distilled water, incubated for 10 min in 10 mM sodium citrate buffer containing 10 mM EDTA, 0.1 mg/ml tetramethyl benzidine and 0.001% hydrogen peroxide, pH 5. The visual intensity of the blue spots which developed on the membrane was determined subjectively, then correlated with the DNA content of the original sample. The results were as shown in Table 6.

**Table 6**

| VISUAL DETECTION OF DNA USING SSB-HRP | |
|---|---|
| pg of DNA in Insulin Sample | Intensity of Color |
| 40 | ++++ |
| 20 | ++ |
| 10 | + |
| 5 | slight positive |
| 0 | - |

### Example 7

### Detection of DNA Using Specific Capture of Sample DNA on an Anti-Biotin Membrane

### A. Purification of SSB-Urease Conjugate

In this example, SSB-urease was further purified to remove unconjugated urease by gel filtration chromatography of the SSB-urease on a column of 1 cm x 30 cm of Superose 6HR (Pharmacia, Piscataway, NJ) in a buffer containing 50 mM sodium sulfite, 20 mM sodium dihydrogen phosphate, 200 mM sodium chloride, 1 mM EDTA, 2 mM dithiothreitol, 0.1% Tween-20 pH 7.00. The protein peak eluting before the unconjugated urease peak was collected and combined with glycerol 1:1 (v:v) and stored at -20°C.

### B. Biosensor Detection of DNA in Buffer with SSB-Urease Conjugate (Sequential Addition of Reagents)

Anti-biotin-coated nitrocellulose membranes prepared as described in Example 4A, were coated with biotin-anti-DNA by filtering 200 »l of biotin-anti-DNA (300 ng/ml in 10 mM TRIS-HCl containing 1% BSA, 1 mM EDTA) prepared as described above in Example 1E through the antibiotin membrane over a period of about 4 min. The membranes were then washed with 300 »l PBS. 200 »l of the samples containing DNA (0, 12, 25, 50 pg/200 »l DNA in PBS) were filtered over a period of about 4 min. The filter was again washed with 300 »l of PBS. Over about 4 min, 200 »l of SSB-urease conjugate, purified as described in Example 7A, (100 ng/ml diluted in 2% BSA, 2% Ficoll®, 2% polyvinylpyrrolidone, 10 mM sodium phosphate, 40 mM NaCl 2 mM EDTA, pH 7.5) was filtered through the membrane. The membrane was then washed 3 times with 1 ml of 1 mM sodium acetate, 0.1 M NaCl, 0.05% Tween-20, pH 5. The membrane was added to substrate solution (acetate wash buffer containing 100 mM urea) and the rate of product formation measured as described above in Example 3B. The rate of product formation was a function of the concentration of DNA in the sample, as shown in Table 7.

**Table 7**

| BIOSENSOR DETECTION OF DNA USING B-UREASE CONJUGATE | |
|---|---|
| Amount of DNA (pg) | Rate (uV/sec) |
| 50 | 546 |
| 25 | 300 |
| 12 | 183 |
| 0 | 120 |

### C. Titration of Biotin-Anti-DNA for a Simultaneous-Incubation Assay

The effect of simultaneously adding biotin-anti-DNA and SSB-urease conjugate purified as described in Example 7A to the nitrocellulose membrane was assessed as follows:

200 »l of sample (1% BSA in TE with or without 100 pg ssDNA) was incubated with 200 »l biotin-anti-DNA (40, 81, 162, 325 ng/ml in 1% BSA, TE) and 200 »l SSB-urease conjugate (15 ng/ml in 2% BSA, 2% Ficoll®, 2% polyvinylpyrrolidone, 10 mM sodium phosphate, 40 mM NaCl, 2 mM EDTA, pH 7.5) at room temperature for 50 min. The mixture was then filtered slowly over a period of about 10 min through a nitrocellulose membrane. The membrane was then washed 3 times with 1 ml of acetate wash buffer (1 mM sodium acetate, 0.1 M NaCl, 0.05% Tween-20, pH 5). Substrate solution (acetate wash buffer containing 100 mM urea) was added and the amount of DNA present on the membrane determined using the biosensor procedure as described above in Example 3B. The results, shown in Table 8 below, show that in a simultaneous assay, the ratio of biotin-anti-DNA and SSB-urease conjugate is critical, presumably due to competition of both anti-DNA and SSB for DNA.

**Table 8**

| TITRATION OF BIOTIN-ANTI-DNA THROUGH SIMULTANEOUS-INCUBATION ASSAY | | |
|---|---|---|
| Concentration of Biotin-Anti-DNA (ng/ml) | Rate (uV/sec) | |
| | 100 pg DNA | No DNA |
| 325 | 308 | 108 |
| 162 | 476 | 149 |
| 81 | 1046 | 238 |
| 40 | 1308 | 217 |

### Example 8.

### Biosensor Detection of DNA in Granulocyte Macrophage Colony Stimulating Factor (GMCSF) Samples with SSB-HRP Conjugate (Sequential Addition)

300 »l of recombinant human GMCSF 3.4 mg/ml PBS was spiked with 0, 5, 10, 50, or 100 pg double-stranded calf thymus DNA. The samples were heated to 100°C for 5 min to denature the DNA, then cooled to room temperature. Immobilon™ membranes (Millipore, Bedford, MA) were coated with goat anti-biotin IgG (Sigma, St Louis, MO), as described above for SSB coating on Immobilon™ in Example 2A. 200 »l of biotin-labeled anti-DNA (clone 4H2) was filtered through the membrane. 300 »l of the samples containing GMCSF were filtered through the membranes and the membranes washed again with 200 »l of PBS. 200»l of SSB-HRP conjugate (650 ng/ml, diluted in PBS containing 0.1 mg/ml BSA, 2 mM EDTA, 5% Triton-X 100) was filtered through the membrane. Membranes were then washed once with PBS containing 5% Triton-X 100, 1 M urea, and washed with 0.1 M sodium acetate, containing 5% ethanol and 1 mM EDTA, pH 5.5. The amount of DNA was then determined by the biosensor method as described above in US Patent No. 4,591,550, which disclosure is incorporated herein by reference. The substrate solution comprised sodium acetate wash buffer containing 250 »M tetramethyl benzidine, 50 »M ruthenium, 500 »M hydrogen peroxide, pH 5.5. The change in potential due to the HRP redox reaction resulted in a change in the signal of the photoresponsive device. The results were as shown in Table 9, below.

**Table 9**

| BIOSENSOR DETECTION OF DNA IN GMCSF SAMPLES USING SSB-HRP CONJUGATE | |
|---|---|
| pg of DNA in 1 mg GMCSF (1 mg/ml??) | Rate (uV/sec) |
| 0 | -137 |
| 5 | -201 |
| 10 | -268 |
| 50 | -629 |
| 100 | -1550 |

### Example 9

### Detection of Bacteria in a Water Sample

Cultures of gram-negative and gram-positive bacteria are diluted in 100 »l HPLC-grade water to 80, 400, 2,000, 10,000/100 »l. For one set of samples, the bacteria are lysed and denatured by treatment with 10 »l of 3N NaOH, then neutralized with 50 »l of 1 M TRIS-HCl, pH 7.3. A second identical set of dilutions was not so treated and serve as a control for non-specific interaction between the sample and the SSB-HRP conjugate.

Both sets of dilutions are loaded onto a positively charged nylon membrane (Genatran™) using a dot-blot apparatus (Schleiker and Schuell). Each well of the apparatus is loaded with 165 »l of sample. Standards, consisting of denatured calf thymus DNA diluted in HPLC-grade water) are also prepared and loaded onto the positively charged nylon membranes. Standards contain 0, 5, 10, or 50 pg ssDNA. DNA is detected using the visual assay as described above in Example 3A using an SSB-HRP conjugate.

The subject methods and compositions provide a rapid and simple means for detecting picogram amounts of DNA in a sample by the use of high affinity single-stranded DNA binding proteins. The assay is applicable not only to pure DNA samples but may also be used with samples which contain a significant amount of protein, or for detecting contamination of a sample with a microorganism.

All publications and patent applications mentioned in this specification are indicative of the level of skill of those skilled in the art to which this invention pertains.

## Claims

1. A method for detecting DNA in a sample using a high affinity single-stranded DNA binding protein (BP) wherein the BP or the DNA comprises a detectable label, said method comprising:
contacting any single-stranded DNA in the sample with the BP to form DNA-BP complexes;
freeing the complexes of unbound sample and BP; and
detecting the complexes by means of the label as indicative of the presence of DNA in the sample.

2. A method according to claim 1, wherein the sample is subjected to DNA denaturing conditions prior to the contacting.

3. A method according to claim 1 or claim 2, wherein the DNA-BP complex is on a solid support.

4. A method according to claim 3 wherein the detectable label is attached to a photoreactive compound and the method includes
admixing the sample with the photoreactive compound;
irradiating the mixture with activating light to react the activated compound with any DNA present in the sample;
contacting the irradiated mixture with the solid support; and
detecting any photoreactive compound present on the solid support by means of the label as indicative of the presence of DNA in the sample.

5. A method according to claim 4, wherein the photoreactive compound is isopsoralen.

6. A method according to claim 5, wherein the detectable label is biotin.

7. A method according to claim 6, wherein the biotin is detected using a conjugate comprising avidin, streptavidin or antibiotin joined to an enzyme, radioisotope or fluorophore.

8. A method according to claim 3, wherein the solid support is an anti-biotin coated membrane.

9. A method according to claim 1, wherein prior to said contacting, the sample is (a) subjected to DNA denaturing conditions; and (b) the denatured DNA is bound non-diffusively to a solid support.

10. A method according to claim 9, which includes subsequently carrying out the contacting step by
contacting the solid support with BP to form DNA-BP complexes; and
carrying out the freeing step by freeing the solid support of any unbound sample and BP.

11. A method according to claim 10, wherein the solid support is a positively charged nylon membrane or a nitrocellulose membrane.

12. A method according to claim 1, wherein the BP is bound non-diffusively to a solid support.

13. A method according to claim 1, wherein the BP is E. coli single-stranded DNA binding protein (SSB), T4 gene 32 protein or topoisomerase I.

14. A method according to claim 1, wherein the label is an enzyme joined to the BP.

15. A method according to claim 14, wherein the enzyme is urease or horseradish peroxidase.

16. A method according to claim 1 further comprising:
additionally treating at least one background solution containing no DNA and at least one reference solution containing a known amount of DNA according to the method of claim 1;
subtracting the label detected in the background solution from the label detected in the sample and from the label detected in said reference solution; and
relating the adjusted label detected in the sample and the reference solution to determine the amount of DNA present in the sample.

17. A method according to claim 1, wherein the sample further includes protein.

18. A method according to claim 17, wherein the protein-containing sample is deproteinized prior to the contacting.

19. A method according to claim 18, wherein the protein-containing sample is deproteinized by a method comprising:
admixing the protein-containing sample with (a) a suspension comprising glass particles and (b) sufficient iodide to cause the DNA to bind to the glass particles;
isolating the glass particles; and
eluting any DNA from the particles.

20. A method according to claim 19, wherein the eluting is with water or phosphate buffered saline.

21. A method according to claim 17, wherein the protein-containing sample is deproteinized by a method comprising:
admixing the protein-containing sample with a proteolytic enzyme composition under conditions whereby protein present is hydrolyzed.

22. A method according to claim 21, further comprising:
after the admixing, separating any product of the hydrolyzed protein from any DNA.

23. A method according to claim 22, wherein the separating comprises:
after the admixing, centrifuging the sample through a membrane with a low molecular weight cutoff; or passing the sample through an ultrafiltration device or a gel permeation chromatography matrix with a low molecular weight cutoff; and
recovering any DNA present.

24. A method according to claim 21, wherein the proteolytic enzyme composition includes pronase and/or proteinase K.

25. A conjugate comprising a high affinity single-stranded DNA binding protein (BP) covalently joined to an enzyme.

26. A conjugate according to claim 25, wherein the BP is E. coli single-stranded DNA binding protein (SSB), T4 gene 32 protein, or topoisomerase I.

27. A conjugate according to claim 26, wherein the enzyme is urease or horseradish peroxidase.

28. A method for detecting an organism in a sample using a DNA-detection system having as a first component a high affinity single-stranded DNA binding protein (BP); wherein at least one component of the system comprises a label; and wherein single-stranded DNA (ssDNA) prepared from the organism or at least one component of the system is bound non-diffusively to a solid support, the method comprising:
contacting the ssDNA with the first component to form ssDNA-BP complexes;
freeing the complexes of unbound ssDNA and components of the system; and
detecting the complexes by means of the label as indicative of the presence of an organism in the sample.

29. A method according to claim 28, wherein the label is conjugated to the first component.

30. A method according to claim 28, wherein the organism is a bacterium, a yeast cell or a virus.

31. A method according to claim 30, wherein the bacterium is a gram-negative bacterium.

32. A method according to claim 30, wherein the gram-negative bacterium is E. coli.

33. A method according to claim 30, further comprising:
prior to the contacting, exposing the cell to a cell-disruptive composition in an amount and for a time sufficient to release the DNA from the cell; and
denaturing the DNA to single-stranded DNA (ssDNA).

## Patentansprüche

1. Verfahren zum Nachweisen von DNA in einer Probe unter Verwendung eines einstrangigen DNA-bindenden Proteins (BP) mit hoher Affirität, worin das BP oder die DNA eine nachweisbare Markierung enthält, wobei das genannte Verfahren umfaßt:
das In-Kontakt-Bringen jeglicher einstrangiger DNA in der Probe mit dem BP, um DNA-BP-Komplexe zu bilden;
das Befreien der Komplexe von ungebundener(em) Probe und BP und
das Nachweisen der Komplexe durch die Markierung als Indikator für das Vorhandensein von DNA in der Probe.

2. Verfahren nach Anspruch 1, worin die Probe vor dem In-Kontakt-Bringen DNA-denaturierenden Bedingungen unterworfen wird.

3. Verfahren nach Anspruch 1 oder 2, worin der DNA-BP-Komplex auf einem festen Träger vorliegt.

4. Verfahren nach Anspruch 3, worin die nachweisbare Markierung an eine photoreaktive Verbindung gebunden ist und das Verfahren umfaßt:
das Mischen der Probe mit der photoreaktiven Verbindung;
das Bestrahlen der Mischung mit aktivierendem Licht, um die aktivierte Verbindung mit jeglicher in der Probe vorhandener DNA umzusetzen;
das In-Kontakt-Bringen der bestrahlten Mischung mit dem festen Träger; und
das Nachweisen jeglicher auf dem festen Träger vorhandener photoreaktiver Verbindung durch die Markierung als Indikator für das Vorhandensein von DNA in der Probe.

5. Verfahren nach Anspruch 4, worin die photoreaktive Verbindung Isopsoralen ist.

6. Verfahren nach Anspruch 5, worin die nachweisbare Markierung Biotin ist.

7. Verfahren nach Anspruch 6, worin das Biotin unter Verwendung eines Konjugats nachgewiesen wird, das mit einem Enzym, Radioisotop oder Fluorophor verbundenes Avidin, Streptavidin oder Antibiotin umfaßt.

8. Verfahren nach Anspruch 3, worin der feste Träger eine mit Antibiotin beschichtete Membran ist.

9. Verfahren nach Anspruch 1, worin die Probe vor dem genannten In-Kontakt-Bringen (a) DNA-denaturierenden Bedingungen ausgesetzt wird; und (b) die denaturierte DNA nicht-diffusiv an einen festen Träger gebunden wird.

10. Verfahren nach Anspruch 9, welches die darauffolgende Durchführung des Schritts des In-Kontakt-Bringens durch
In-Kontakt-Bringen des festen Trägers mit BP, um DNA-BP-Komplexe zu bilden; und
Durchführen des Befreiungsschrittes durch Befreien des festen Trägers von jeglicher/-em ungebundener/-en Probe und BP umfaßt.

11. Verfahren nach Anspruch 10, worin der feste Träger eine positiv geladene Nylonmembran oder eine Nitrozellulosemembran ist.

12. Verfahren nach Anspruch 1, worin das BP nicht-diffusiv an einen festen Träger gebunden wird.

13. Verfahren nach Anspruch 1, worin das BP einstrangiges E.coli-DNA-bindendes Protein (SSB), T4 Gen 32-Protein oder Topoisomerase I ist.

14. Verfahren nach Anspruch 1, worin die Markierung ein mit dem BP verbundenes Enzym ist.

15. Verfahren nach Anspruch 14, worin das Enzym Urease oder Meerrettichperoxidase ist.

16. Verfahren nach Anspruch 1, das weiters umfaßt:
die zusätzliche Behandlung zumindest einer Hintergrundlösung, die keine DNA enthält, und zumindest einer Bezugslösung, die eine bekannte Menge an DNA enthält, nach dem Verfahren nach Anspruch 1;
das Subtrahieren der in der Hintergrundlösung festgestellten Markierung von der in der Probe festgestellten Markierung und von der in der genannten Bezugslösung festgestellten Markierung; und
das In-Beziehung-Setzen der in der Probe und in der Bezugslösung festgestellten berichtigten Markierung, um die in der Probe vorhandene DNA-Menge zu bestimmen.

17. Verfahren nach Anspruch 1, worin die Probe weiters Protein enthält.

18. Verfahren nach Anspruch 17, worin die proteinhältige Probe vor dem In-Kontakt-Bringen entproteinisiert wird.

19. Verfahren nach Anspruch 18, worin die proteinhältige Probe nach einem Verfahren entproteinisiert wird, das umfaßt:
das Mischen der proteinhältigen Probe mit (a) einer Glasteilchen enthaltenden Suspension und (b) ausreichend Jodid, um zu bewirken, daß die DNA sich an die Glasteilchen bindet;
das Isolieren der Glasteilchen; und
das Eluieren jeglicher DNA aus den Teilchen.

20. Verfahren nach Anspruch 19, worin das Eluieren mit Wasser oder phosphatgepufferter Salzlösung erfolgt.

21. Verfahren nach Anspruch 17, worin die proteinhältige Probe nach einem Verfahren entproteinisiert wird, das umfaßt:
das Mischen der proteinhältigen Probe mit einer proteolytischen Enzymzusammensetzung unter Bedingungen, bei denen vorhandenes Protein hydrolysiert wird.

22. Verfahren nach Anspruch 21, das weiters umfaßt:
nach dem Mischen das Abtrennen jeglichen Produkts des hydrolysierten Proteins von jeglicher DNA.

23. Verfahren nach Anspruch 22, worin das Abtrennen umfaßt:
nach dem Mischen das Zentrifugieren der Probe durch eine Membran mit Niedrigmolekulargewichts-Cutoff; oder das Hindurchschicken der Probe durch eine Ultrafiltrationsvorrichtung oder eine Gelpermeationschromatographiematrix mit Niedrigmolekulargewichts-Cutoff; und
das Wiedergewinnen jeglicher vorhandener DNA.

24. Verfahren nach Anspruch 21, worin die proteolytische Enzymzusammensetzung Pronase und/oder Proteinase K enthält.

25. Konjugat, das ein einstrangiges DNA-bindendes Protein (BP) mit hoher Affinität umfaßt, das kovalent an ein Enzym gebunden ist.

26. Konjugat nach Anspruch 25, worin das BP einstrangiges E.coli-DNA-bindendes Protein (SSB), T4 Gen 32-Protein oder Topoisomerase I ist.

27. Konjugat nach Anspruch 26, worin das Enzym Urease oder Meerrettichperoxidase ist.

28. Verfahren zum Nachweisen eines Organismus in einer Probe unter Verwendung eines DNA-Nachweissystems, das als eine erste Komponente ein einstrangiges DNA-bindendes Protein (BP) mit hoher Affinität aufweist; worin zumindest eine Komponente des Systems eine Markierung enthält; und worin aus dem Organismus hergestellte einstrangige DNA (ssDNA) oder zumindest eine Komponente des Systems nichtdiffusiv an einen festen Träger gebunden wird, wobei das Verfahren umfaßt:
das In-Kontakt-Bringen der ssDNA mit der ersten Komponente, um ssDNA-BP-Komplexe zu bilden;
das Befreien der Komplexe von ungebundener ssDNA und Komponenten des Systems; und
das Nachweisen der Komplexe durch die Markierung als Indikator für das Vorhandensein eines Organismus in der Probe.

29. Verfahren nach Anspruch 28, worin die Markierung an die erste Komponente konjugiert ist.

30. Verfahren nach Anspruch 28, worin der Organismus ein Bakterium, eine Hefezelle oder ein Virus ist.

31. Verfahren nach Anspruch 30, worin das Bakterium ein gramnegatives Bakterium ist.

32. Verfahren nach Anspruch 30, worin das gramnegative Bakterium E.coli ist.

33. Verfahren nach Anspruch 30, das weiters umfaßt:
vor dem In-Kontakt-Bringen das Aussetzen der Zelle an eine zellaufbrechende Zusammensetzung in einer Menge und für einen Zeitraum, die ausreichen, um die DNA aus der Zelle freizusetzen; und das Denaturieren der DNA zu einstrangiger DNA (ssDNA).

## Revendications

1. Méthode de détection d'ADN dans un échantillon utilisant une protéine liante (BP) d'ADN à brin unique à haute affinité dans laquelle le BP ou l'ADN comprend un marqueur détectable, ladite méthode comprenant :
la mise en contact d'un ADN à brin unique dans l'échantillon avec le BP pour former des complexes d'ADN-BP;
la libération des complexes de l'échantillon non lié et du BP; et
la détection des complexes au moyen du marqueur en tant qu'indicateur de la présence de l'ADN dans l'échantillon.

2. Méthode selon la revendication 1, dans laquelle l'échantillon est soumis à des conditions de dénaturation d'ADN avant la mise en contact.

3. Méthode selon la revendication 1 ou 2, dans laquelle le complexe ADN-BP est sur un support solide.

4. Méthode selon la revendication 3, dans laquelle le marqueur détectable est lié à un composé photoréactif et la méthode inclut :
le mélange de l'échantillon avec le composé photoréactif;
l'irradiation du mélange avec une lumière activante pour faire réagir le composé activé avec un ADN présent dans l'échantillon;
la mise en contact du mélange irradié avec le support solide; et
la détection de tout composé photoréactif présent sur le support solide au moyen du marqueur en tant qu'indication de la présence d'ADN dans l'échantillon.

5. Méthode selon la revendication 4, dans laquelle le composé photoréactif est l'isopsoralène.

6. Méthode selon la revendication 5, dans laquelle le marqueur détectable est la biotine.

7. Méthode selon la revendication 6, dans laquelle la biotine est détectée en utilisant un conjugué comprenant de l'avidine, de la pétravidine ou de l'anti-biotine jointe à un enzyme, un radio-isotope ou un fluorophore.

8. Méthode selon la revendication 3, dans laquelle le support solide est une membrane recouverte d'anti-biotine.

9. Méthode selon la revendication 1, dans laquelle avant ladite mise en contact, l'échantillon est (a) soumis à des conditions dénaturantes d'ADN; et (b) l'ADN dénaturé est lié de façon non diffusive à un support solide.

10. Méthode selon la revendication 9, qui inclut la mise en oeuvre subséquente de l'étape de mise en contact par
la mise en contact du support solide avec BP pour former des complexes ADN-BP; et
la mise en oeuvre de l'étape de libération par libération du support solide de tout échantillon non lié et BP.

11. Méthode selon la revendication 10, dans laquelle le support solide est une membrane de nylon chargée positivement ou une membrane de nitrocellulose.

12. Méthode selon la revendication 1, dans laquelle le BP est lié de façon non diffusive à un support solide.

13. Méthode selon la revendication 1, dans laquelle le BP est une protéine liante d'ADN à brin unique de E. Coli (SSB), une protéine T4 du gène 32 ou une topoisomérase I;

14. Méthode selon la revendication 1, dans laquelle le marqueur est un enzyme joint au BP.

15. Méthode selon la revendication 14, dans laquelle l'enzyme est une uréase ou une peroxydase de raifort.

16. Méthode selon la revendication 1, comprenant de plus :
le traitement additionnel d'au moins une solution de fond ne contenant pas d'ADN et d'au moins une solution de référence contenant une quantité connue d'ADN selon la méthode de la revendication 1;
la soustraction du marqueur détecté dans la solution de fond du marqueur détecté dans l'échantillon et du marqueur détecté dans ladite solution de référence; et
la mise en relation du marqueur ajusté détecté dans l'échantillon et la solution de référence pour déterminer la quantité d'ADN présente dans l'échantillon.

17. Méthode selon la revendication 1, dans laquelle l'échantillon inclut de plus une protéine.

18. Méthode selon la revendication 17, dans laquelle l'échantillon contenant une protéine est déproténéisé avant la mise en contact.

19. Méthode selon la revendication 18, dans laquelle l'échantillon contenant une protéine est déproténéisé par une méthode comprenant :
l'ajout de l'échantillon contenant une protéine avec (a) une suspension comprenant des particules de verre et (b) suffisamment d'iodure pour faire que l'ADN se lie aux particules de verre;
l'isolation des particules de verre; et
l'élution de tout ADN des particules.

20. Méthode selon la revendication 19, dans laquelle l'élution est effectuée avec de l'eau ou une solution saline tamponnée phosphate.

21. Méthode selon la revendication 17, dans laquelle l'échantillon contenant une protéine est déproténéisé par une méthode comprenant :
le mélange de l'échantillon contenant une protéine avec une composition d'enzymes protéolytiques dans des conditions dans lesquelles la protéine présente est hydrolysée.

22. Méthode selon la revendication 21, comprenant de plus
après le mélange, la séparation de tout produit de la protéine hydrolysée de tout ADN.

23. Méthode selon la revendication 22, dans laquelle la séparation comprend :
après le mélange, la centrifugation de l'échantillon à travers une membrane avec une coupure de poids moléculaires faibles; ou le passage de l'échantillon par un dispositif d'ultrafiltration ou une matrice de chromatographie par perméation de gel avec une coupure de poids moléculaires faibles; et
la récupération de tout ADN présent.

24. Méthode selon la revendication 21, dans laquelle la composition d'enzymes protéolytiques inclut une pronase et/ou une protéinase K.

25. Conjugué comprenant une protéine liante (BP) d'ADN à brin unique à affinité élevée liée de façon convalente à un enzyme.

26. Conjugué selon la revendication 25, dans lequel le BP est une protéine Plante d'ADN à brin unique de E. Coli, une protéine T4 de gène 32 (SSB), ou une topoisomérase I.

27. Conjugué selon la revendication 26, dans lequel l'enzyme est une uréase ou une peroxydase de raifort.

28. Méthode pour détecter un organisme dans un échantillon en utilisant un système de détection d'ADN ayant en tant que premier composant une protéine liante (BP) d'ADN à brin unique à affinité élevée, dans laquelle au moins un composant du système comprend un marqueur; et dans laquelle l'ADN à brin unique (ssADN) préparé à partir de l'organisme ou au moins un composant du système est lié de façon non diffusive à un support solide, la méthode comprenant :
la mise en contact du ssADN avec le premier composant pour former des complexes ssADN-BP;
la libération des complexes du ssADN non liés et des composant's du système; et
la détection des complexes au moyen du marqueur en tant qu'indicateurs de la présence d'un organisme dans l'échantillon.

29. Méthode selon la revendication 28, dans laquelle le marqueur est conjugué au premier composant.

30. Méthode selon la revendication 28, dans laquelle l'organisme est une bactérie, une cellule de levure ou un virus.

31. Méthode selon la revendication 30, dans laquelle la bactérie est une bactérie gram-négative.

32. Méthode selon la revendication 30, dans laquelle la bactérie gram-négative est E. Coli.

33. Méthode selon la revendication 30, comprenant de plus :
avant la mise en contact, l'exposition de la cellule à une composition rompant la cellule en une quantité et pendant un temps suffisant pour libérer l'ADN de la cellule; et
la dénaturation de l'ADN en ADN en brin unique (ssADN).
